# EUROPEAN PATENT SPECIFICATION

(11) **EP 3 380 145 B1**
(45) Date of publication and mention of the grant of the patent: **13.11.2019**
(21) Application number: 16798204.0
(22) Date of filing: 21.11.2016
(51) Int. Cl.: A61M 5/24, A61M 5/28, A61M 5/315, A61M 5/178

(54) **INJECTION APPARATUS**
INJEKTIONSVORRICHTUNG
APPAREIL D'INJECTION

(30) Priority: 27.11.2015 EP 15196703
(43) Date of publication of application: 03.10.2018
(73) Proprietor: Sanofi-Aventis Deutschland GmbH, 65929 Frankfurt am Main (DE)
(72) Inventor: DITTRICH, Marcus-Meinolf, 65510 Idstein (DE)
(74) Representative: Derry, Paul Stefan
(86) International application number: PCT/EP2016/078269
(87) International publication number: WO 2017/089280

(56) References cited:
- WO-A2-2004/037326
- FR-A1- 2 573 310
- GB-A- 180 215
- GB-A- 1 334 219
- US-A- 2 735 429
- US-A- 3 380 449
- US-A- 3 391 695
- US-A1- 2005 267 403

## Description

### Field

The present invention relates to injection apparatus containing liquid medicament.

### Background

Pre-filled injection apparatus typically fall into two categories: manual apparatus and auto-injectors. In a conventional manual apparatus, a user must provide force to drive a medicament through a needle. This is typically done by some form of button / plunger that has to be continuously pressed during the injection. Both categories have in common that the medicament reservoir is pre-filled with the medicament at the production site so that a user does not have to undertake injection preparation steps such as drawing the desired amount of medicament from a vial into the injection device reservoir.

One example for this is a pre-filled syringe, where medicament is stored in a hollow cylinder, typically formed of glass. For the duration of the shelf life, the medicament is sealed from the environment with a plunger moveable within the cylinder, and a needle fluidly connected to the syringe's distal end. The needle must remain capped in order to maintain the medicament under sterile conditions.

Another form of injection device uses a cartridge instead of a syringe, the cartridge having a distal seal instead of the syringe's needle. Typically a patient connects a double-ended needle to the cartridge before injection, thereby piercing the cartridge's seal with the proximal tip of the double-ended needle.

Auto-injectors make self-injection easier for patients. A conventional autoinjector may provide the force for administering the injection by a spring, and a trigger button or other mechanism may be used to activate or initiate the injection. Auto-injectors may be single-use or reusable apparatus. Auto-injectors may be single-dose, delivering the entire contents of a cartridge or pre-filled syringe, or may be fixed-dose, delivering predetermined amounts from the cartridge or pre-filled syringe. A reusable auto-injector utilizes disposable safety needles and cartridges or pre-filled syringes.

While a cartridge can provide storage advantages relative to syringes, they are not without shortcomings. For example, the attachment of a needle to the cartridge requires an additional step. This step can be problematic for patients with limited dexterity, poor coordination, or who have lost a degree of sensation in their hands.

Even with such disadvantages, in certain situations it is desirable to provide an injection device in which the needle is kept separate from the medicament until such time as the patient wishes to commence the injection, especially in cases where the medicament formulation is sensitive to a long time exposure to the metal material that forms the injection needle. The injection device described herein aims to overcome one or more problems associated with prior devices.

### Summary

In one aspect, an injection apparatus is provided. The injection apparatus comprises a hollow body having a first end at which a hollow needle is or can be located. A bung with a rupture seal membrane is located within the hollow body. A piston is also located within the hollow body. The piston, the hollow body and the bung define a volume containing a liquid medicament. The bung is nearer than the piston to the first end of the hollow body, and the rupture seal membrane initially isolates the needle from the medicament. The rupture seal membrane is configured to rupture through fluid pressure alone, without the rupture seal membrane contacting the needle, thereby to allow the medicament to be expelled through the needle upon movement of the piston causing the provision of pressure to the medicament. The piston comprises a protrusion located on the side of the piston which faces the first end of the hollow body, the protrusion shaped so as to extend into an aperture in the bung at the end of medicament expulsion.

The needle may be connected at the first end of the hollow body and an end of the needle that is internal to the hollow body may be nearer than the rupture seal membrane to the first end of the hollow body.

The aperture in the bung may be circular and, if so, the protrusion of the piston will be cylindrical in shape.

The aperture in the bung may lie on the longitudinal axis of the bung and, if so, the protrusion of the piston will lie on the longitudinal axis of the piston.

The diameter of the aperture in the bung may be the same as an outside diameter of the protrusion.

The length of the protrusion may be the same or substantially the same as the length of the aperture in the bung.

The bung may comprise at least two oppositely oriented rupture seal membranes, wherein at least one of the rupture seal membranes is configured to rupture under pressure from a first side of the bung and wherein at least one of the rupture seal membranes is configured to rupture under pressure from the other side of the bung.

The one or more rupture seal membranes may be convex when in an unstressed position.

The injection apparatus may be a medicament cartridge for use in an injector.

A liquid substance may be disposed between the first end of the hollow body and the bung.

The hollow body may include a neck and the bung may be located against the neck.

The injection apparatus may be a syringe device and the hollow body may form the outer housing of the syringe device.

The bung may be located against the first end of the hollow body.

Another aspect provides a method of constructing an injection apparatus. The method comprises:
supporting a hollow body, the hollow body comprising a front end configured to house a hollow needle and a rear end which is open;
inserting a bung into the hollow body at its rear end, wherein the bung comprises a rupture seal membrane configured to rupture through fluid pressure alone;
pushing the bung along the hollow body towards its front end, the bung forming a seal with an inner wall of the hollow body;
providing the hollow body with a liquid medicament;
inserting a piston into the hollow body at its rear end; and
pushing the piston along the hollow body towards the bung, until the piston reaches the medicament, wherein the piston forms a seal with the inner wall of the hollow body thereby sealing the medicament in the volume defined by the piston, the hollow body and the bung.

A further aspect provides Injection apparatus. The injection apparatus comprises a hollow body having a first end at which a hollow needle is or can be located. It also comprises a bung located within the hollow body. The bung comprises at least two oppositely oriented rupture seal membranes. At least one of the rupture seal membranes is configured to rupture under fluid pressure from a first side of the bung and wherein at least one of the rupture seal membranes is configured to rupture under fluid pressure from the other side of the bung. A piston is located within the hollow body, and the piston, the hollow body and the bung define a volume containing a liquid medicament. The bung is nearer than the piston to the first end of the hollow body. The rupture seal membrane initially isolates the needle from the medicament.

At least one of the rupture seal membranes is curved in an opposite direction to at least one other of the rupture seal membranes.

The one or more rupture seal membranes may be convex when in an unstressed position.

The needle may be connected at the first end of the hollow body and an end of the needle that is internal to the hollow body may be nearer than the rupture seal membrane to the first end of the hollow body.

The injection apparatus may be a medicament cartridge for use in an injector.

A liquid substance may be disposed between the first end of the hollow body and the bung.

The hollow body may include a neck and the bung may be located against the neck.

The injection apparatus may be a syringe device and the hollow body may form the outer housing of the syringe device.

The bung may be located against the first end of the hollow body.

Yet another aspect provides a method of constructing an injection apparatus. The method comprises:
supporting a hollow body, the hollow body comprising a front end configured to house a hollow needle and a rear end which is open;
inserting a bung into the hollow body at its rear end, wherein the bung comprises at least two oppositely oriented rupture seal membranes, wherein at least one of the rupture seal membranes is configured to rupture under fluid pressure from a first side of the bung and wherein at least one of the rupture seal membranes is configured to rupture under fluid pressure from the other side of the bung;
pushing the bung along the hollow body towards its front end, the bung forming a seal with an inner wall of the hollow body;
providing the hollow body with a liquid medicament;
inserting a piston into the hollow body at its rear end; and
pushing the piston along the hollow body towards the bung, until the piston reaches the medicament, wherein the piston forms a seal with the inner wall of the hollow body thereby sealing the medicament in the volume defined by the piston, the hollow body and the bung.

The terms "drug" or "medicament" which are used interchangeably herein, mean a pharmaceutical formulation that includes at least one pharmaceutically active compound.

The term "drug delivery device" shall be understood to encompass any type of device, system or apparatus designed to immediately dispense a drug to a human or non-human body (veterinary applications are clearly contemplated by the present disclosure). By "immediately dispense" is meant an absence of any necessary intermediate manipulation of the drug by a user between discharge of the drug from the drug delivery device and administration to the human or non-human body. Without limitation, typical examples of drug delivery devices may be found in injection devices, inhalers, and stomach tube feeding systems. Again without limitation, exemplary injection devices may include, e.g., syringes, autoinjectors, injection pen devices and spinal injection systems.

### Brief description of the drawings

Exemplary embodiments of the present invention are described with reference to the accompanying drawings, in which:
Figures 1A and 1B are isometric views of an injection device;
Figure 2 is a cross sectional view of a cartridge for the Figure 1 injection device;
Figure 3A is an isometric view of a bung shown in the Figure 2 cartridge;
Figure 3B is an isometric view of a piston which can be used with the cartridge of Figure 2;
Figure 3C is an isometric view of the bung and piston of Figures 3A and 3C, in use;
Figure 4A is a cross sectional view of the bung shown in Figure 2;
Figure 4B is a cross sectional view of an alternative bung;
Figure 4C is a plan view of another alternative bung;
Figure 5 is a flow chart for manufacturing the cartridge shown in Figure 2;
Figure 6 is a syringe device and
Figures 7A, 7B and 7C are cross sectional views of an alternative cartridge for the Figure 1 injection device in different stages of use.

### Description

In a first embodiment, a cartridge for an auto-injector is provided. The cartridge is a standard size and shape and is suitable for use in any injector device that accepts standard cartridges of the same type. The cartridge includes a hollow body which holds a liquid medicament. A hollow needle is connected at one end of the hollow body. The hollow body contains a piston or stopper and a bung between which the medicament is contained. The piston and the bung each have substantially the same cross section as the hollow body, so that they both form a seal with the hollow body. In an initial configuration of the cartridge, the bung is located between the medicament and the needle so that it prevents the medicament and the needle from coming into contact. The bung includes a rupture seal membrane which is configured to rupture when the pressure exerted upon it by the medicament is increased as a result of movement of the piston towards the bung. After the rupture seal membrane has ruptured, the medicament can flow to the needle and can then be expelled through the needle and into the patient.

Additionally, a protrusion is added to the side of the piston which faces the bung. The protrusion is shaped so that it extends into the aperture which remains in the bung after the rupture seal membrane has ruptured. This increases (preferably maximises) the quantity of medicament that is delivered to the patient, thereby reducing the amount of wasted medicament, when the piston is pushed the maximum distance, i.e. until it abuts the bung.

The protrusion can take any appropriate shape such that it fits within the aperture. If the aperture is circular, the protrusion is cylindrical. The protrusion may be cylindrical and be positioned centrally on the face of the piston, i.e. on its longitudinal axis, whilst the aperture is circular and positioned on the longitudinal axis of the bung. The rotational symmetry of this arrangement simplifies the manufacture of the cartridge because the bung and piston can be placed within the hollow body at any rotational orientation.

The bung may comprise at least two rupture seal membranes in opposite orientations such that at least one of the rupture seal membranes is configured to rupture under pressure from a first side of the bung and at least one of the rupture seal membranes is configured to rupture under pressure from the other side of the bung. This simplifies the manufacture of the cartridge because the bung can be placed within the hollow body in either orientation.

In a second embodiment, a pre-filled syringe is provided. The pre-filled syringe consists of a hollow body which holds a liquid medicament. A hollow needle is connected at one end of the hollow body. The hollow body contains a piston and a bung between which the medicament is contained. The piston and the bung each have substantially the same cross section as the hollow body, so that they both form a seal with the hollow body. In an initial configuration of the pre-filled syringe, the bung is located between the medicament and the needle so that it prevents the medicament and the needle from coming into contact. The bung includes a rupture seal membrane which is configured to rupture when the pressure exerted upon it by the medicament is increased as a result of movement of the piston towards the bung. After the rupture seal membrane has ruptured, the medicament can flow to the needle and can therefore be expelled through the needle and into the body of the patient. The syringe may include the protrusion feature and/or the oppositely-oriented rupture membranes described in relation to the cartridge.

A drug delivery device, as described herein, may be configured to inject a medicament into a patient. For example, delivery could be sub-cutaneous, intra-muscular, or intravenous. Such a device could be operated by a patient or care-giver, such as a nurse or physician, and can include various types of safety syringe, pen-injector, or auto-injector. The device can include a cartridge-based system that requires piercing a sealed ampule before use. Volumes of medicament delivered with these various devices can range from about 0.5 ml to about 2 ml. Yet another device can include a large volume device ("LVD") or patch pump, configured to adhere to a patient's skin for a period of time (e.g., about 5, 15, 30, 60, or 120 minutes) to deliver a "large" volume of medicament (typically about 2 ml to about 10 ml).

In combination with a specific medicament, the presently described devices may also be customized in order to operate within required specifications. For example, the device may be customized to inject a medicament within a certain time period (e.g., about 3 to about 20 seconds for auto-injectors, and about 10 minutes to about 60 minutes for an LVD). Other specifications can include a low or minimal level of discomfort, or to certain conditions related to human factors, shelf-life, expiry, biocompatibility, environmental considerations, etc. Such variations can arise due to various factors, such as, for example, a drug ranging in viscosity from about 3 cP to about 50 cP. Consequently, a drug delivery device will often include a hollow needle ranging from about 25 to about 31 Gauge in size. Common sizes are 27 and 29 Gauge.

The delivery devices described herein can also include one or more automated functions. For example, one or more of needle insertion, medicament injection, and needle retraction can be automated. Energy for one or more automation steps can be provided by one or more energy sources. Energy sources can include, for example, mechanical, pneumatic, chemical, or electrical energy. For example, mechanical energy sources can include springs, levers, elastomers, or other mechanical mechanisms to store or release energy. One or more energy sources can be combined into a single device. Devices can further include gears, valves, or other mechanisms to convert energy into movement of one or more components of a device.

The one or more automated functions of an auto-injector may each be activated via an activation mechanism. Such an activation mechanism can include one or more of a button, a lever, a needle sleeve, or other activation component. Activation of an automated function may be a one-step or multi-step process. That is, a user may need to activate one or more activation components in order to cause the automated function. For example, in a one-step process, a user may depress a needle sleeve against their body in order to cause injection of a medicament. Other devices may require a multi-step activation of an automated function. For example, a user may be required to depress a button and retract a needle shield in order to cause injection.

In addition, activation of one automated function may activate one or more subsequent automated functions, thereby forming an activation sequence. For example, activation of a first automated function may activate at least two of needle insertion, medicament injection, and needle retraction. Some devices may also require a specific sequence of steps to cause the one or more automated functions to occur. Other devices may operate with a sequence of independent steps.

Some delivery devices can include one or more functions of a safety syringe, pen-injector, or auto-injector. For example, a delivery device could include a mechanical energy source configured to automatically inject a medicament (as typically found in an auto-injector) and a dose setting mechanism (as typically found in a pen-injector).

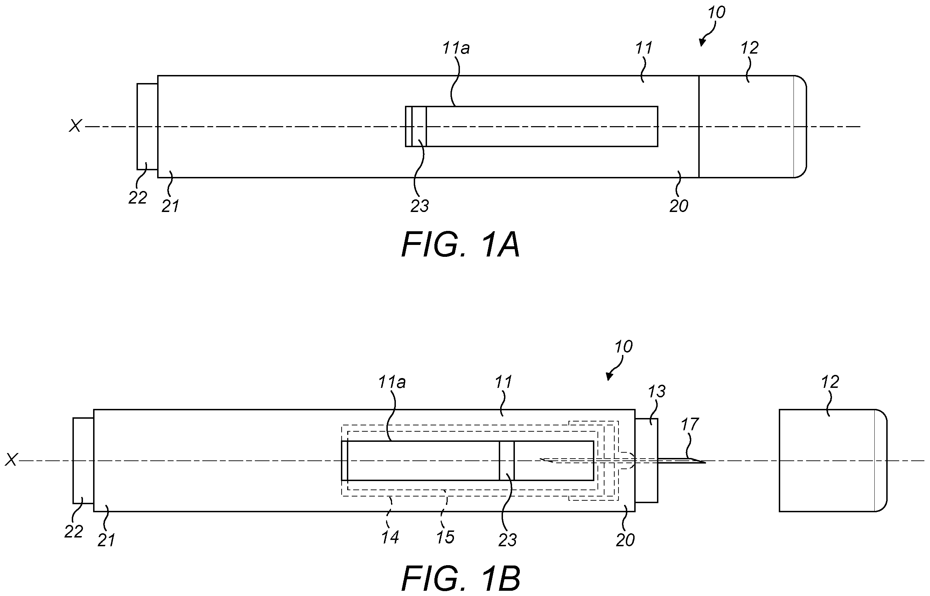

According to some embodiments of the present disclosure, an exemplary drug delivery device 10 is shown in Figs. 1A & 1B. Device 10, as described above, is configured to inject a medicament into a patient's body. Device 10 includes a housing 11 which typically contains a reservoir containing the medicament to be injected (e.g., a syringe) and the components required to facilitate one or more steps of the delivery process. Device 10 can also include a cap assembly 12 that can be detachably mounted to the housing 11. Typically a user must remove cap 12 from housing 11 before device 10 can be operated.

As shown, housing 11 is substantially cylindrical and has a substantially constant diameter along the longitudinal axis X. The housing 11 has a distal region 20 and a proximal region 21. The term "distal" refers to a location that is relatively closer to a site of injection, and the term "proximal" refers to a location that is relatively further away from the injection site.

Device 10 can also include a needle sleeve 13 coupled to housing 11 to permit movement of sleeve 13 relative to housing 11. For example, sleeve 13 can move in a longitudinal direction parallel to longitudinal axis X. Specifically, movement of sleeve 13 in a proximal direction can permit a needle 17 to extend from distal region 20 of housing 11.

Insertion of needle 17 can occur via several mechanisms. For example, needle 17 may be fixedly located relative to housing 11 and initially be located within an extended needle sleeve 13. Proximal movement of sleeve 13 by placing a distal end of sleeve 13 against a patient's body and moving housing 11 in a distal direction will uncover the distal end of needle 17. Such relative movement allows the distal end of needle 17 to extend into the patient's body. Such insertion is termed "manual" insertion as needle 17 is manually inserted via the patient's manual movement of housing 11 relative to sleeve 13.

Another form of insertion is "automated," whereby needle 17 moves relative to housing 11. Such insertion can be triggered by movement of sleeve 13 or by another form of activation, such as, for example, a button 22. As shown in Figs. 1A & 1B, button 22 is located at a proximal end of housing 11. However, in other embodiments, button 22 could be located on a side of housing 11.

Other manual or automated features can include drug injection or needle retraction, or both. Injection is the process by which a bung or piston 23 is moved from a proximal location within a syringe (not shown) to a more distal location within the syringe in order to force a medicament from the syringe through needle 17. In some embodiments, a drive spring (not shown) is under compression before device 10 is activated. A proximal end of the drive spring can be fixed within proximal region 21 of housing 11, and a distal end of the drive spring can be configured to apply a compressive force to a proximal surface of piston 23. Following activation, at least part of the energy stored in the drive spring can be applied to the proximal surface of piston 23. This compressive force can act on piston 23 to move it in a distal direction. Such distal movement acts to compress the liquid medicament within the syringe, forcing it out of needle 17.

Following injection, needle 17 can be retracted within sleeve 13 or housing 11. Retraction can occur when sleeve 13 moves distally as a user removes device 10 from a patient's body. This can occur as needle 17 remains fixedly located relative to housing 11. Once a distal end of sleeve 13 has moved past a distal end of needle 17, and needle 17 is covered, sleeve 13 can be locked. Such locking can include locking any proximal movement of sleeve 13 relative to housing 11.

Another form of needle retraction can occur if needle 17 is moved relative to housing 11. Such movement can occur if the syringe within housing 11 is moved in a proximal direction relative to housing 11. This proximal movement can be achieved by using a retraction spring (not shown), located in distal region 20. A compressed retraction spring, when activated, can supply sufficient force to the syringe to move it in a proximal direction. Following sufficient retraction, any relative movement between needle 17 and housing 11 can be locked with a locking mechanism. In addition, button 22 or other components of device 10 can be locked as required.

A medicament cartridge according to a first embodiment of the present disclosure is a cartridge 200 for an auto-injector. The cartridge consists of a cartridge body 210. The body 210 is hollow, annularly cylindrical in shape and holds a liquid medicament 215 for injection into a patient. The cartridge 200 is rotationally symmetrical about its longitudinal axis. The cartridge body 210 may be transparent and can be made of any suitable material, such as plastic or glass. The shape of the cartridge body 210 is dictated by the auto-injector into which the cartridge 200 will be inserted. Often auto-injectors are configured to receive a cartridge of a certain capacity, e.g. 1ml, 1.5ml or 3ml. It is beneficial for cartridges of a particular capacity to be standardised in their shape and size since such cartridges can be used in any one of many auto-injector device configurations that are designed to accommodate the standard cartridge.

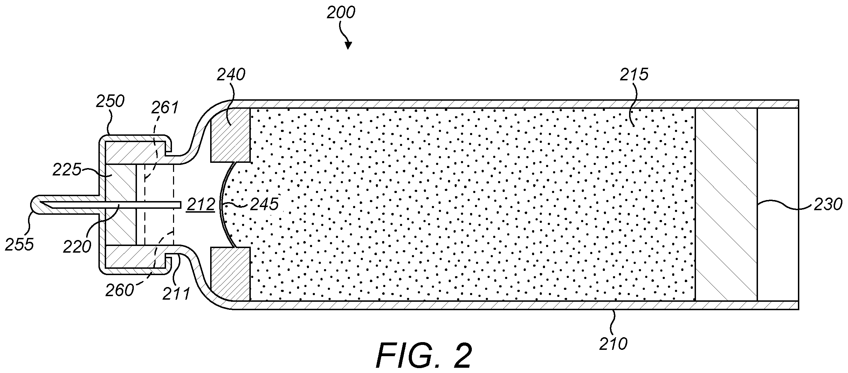

The cartridge 200 is of a standard shape and size. As such, it is usable in injector devices that are designed to accommodate standard cartridges of the same type without modification of the injector device. The cartridge body 210, of the cartridge 200, has a neck 211 near its front end. The diameter of the cartridge body 210 is narrower at the neck 211 than the rest of the cartridge body 210, as can be seen from Figure 2.

A hollow needle 220 is connected to the cartridge body 210 at its front end. The needle 220 can be any suitable needle, and advantageously is of a standard size and shape. The needle 220 is connected to the cartridge body 210 by a septum 225. The septum 225 is held in place by the walls of the cartridge body 210 at its mouth, because the outer diameter on the cross section of the septum 225 is substantially the same as the inner diameter on the cross section of the mouth of the cartridge body 210. Thus the septum 225 forms a seal at the mouth of the cartridge body 210.

An aperture through the centre of the septum 225 is substantially the same diameter as the needle 220. The needle 220 extends perpendicularly through the aperture of the septum 225, such that the needle 220 lies on the longitudinal axis of the cartridge 200 and is held in place by the septum 225. One end of the needle 220 is located inside the cartridge body 210 and is therefore in fluid connection with it. The other end of the needle 220 is located outside of the cartridge body 210 so that it can be inserted into a patient. The end of the needle 220 located outside of the cartridge body has a sharp point which enables it to be easily pushed through the skin of a patient. As seen from Figure 2, approximately equal lengths of the needle 220 may extend from either side of the septum 225.

The cartridge 200 also includes a piston 230, which is contained within the cartridge body 210 and has a cross section which is substantially the same as that of the cartridge body 210. The piston 230 is cylindrical in shape. The piston 230 forms a seal with the walls of the cartridge body 210. The piston 230 can be made of any suitable material, for instance rubber or plastic.

The cartridge 200 also includes a bung 240, which is contained within the cartridge body 210 and has a cross section which is substantially the same as that of the cartridge body 210. The bung 240 is cylindrical in shape, although it is an annular cylinder as described in more detail below. The bung 240 forms a seal with the walls of the cartridge body 210.

The medicament 215 is contained within the volume defined by the cartridge body 210, the piston 230 and the bung 240. The bung 240 is placed closer to the needle 220 than the piston 230, and therefore acts as a barrier, keeping the medicament 215 and the needle 220 isolated from each other.

The volume defined between the bung 240, the septum 225 and the cartridge body 210 is henceforth referred to as the separation volume 212. A separation distance between the needle 220 and the medicament 215 is defined in part by the length of the part of the needle 220 which extends from the septum into the cartridge body 210. In an alternative, the separation volume 212 may be reduced by a septum 225 extending further towards the bung 240 and surrounding the needle end inside the cartridge. This is indicated by the broken lines 260, 261. In this alternative, the inner needle end may be flush with the inner surface of the septum 225.

The piston 230 is configured to be moveable along the longitudinal axis of the cartridge body 210, whilst maintaining the seal with the walls of the cartridge body 210. This could be achieved, for example, by choosing the material of the piston 230 such that the coefficient of friction between it and the walls of the cartridge body 210 is relatively low.

The bung 240 comprises a circular aperture through its centre, such that the bung 240 is annular in shape. The aperture through the centre of the bung 240 is sealed by a rupture seal membrane 245. The rupture seal membrane 245 is made of the same material as the main body of the bung 240 and may be integrally formed therewith. The bung 240 and rupture seal membrane 245 may be a single moulded body. The rupture seal membrane 245 is circular and configured to rupture from fluid pressure alone. The rupture seal membrane 245 does not come into contact with the needle 220. Although the rupture seal membrane 245 stretches partially before rupturing when pressure is exerted upon it, the separation distance is sufficiently large so that the rupture seal membrane 245 ruptures before coming into contact with the needle 220.

The cartridge 200 also comprises a needle holder 250, which is located at the front end of the cartridge body 210 and helps to hold the needle 220 and septum 225 in place. The needle holder 250 is crimped around the neck 211 of the cartridge body 210. This prevents it from moving relative to the cartridge body 210.

The needle holder 250 supports bellows 255 which cover the needle 220 and maintain sterility of the needle. The bellows 255 are flexible so that they flex and come to a stop as they abut the skin of the patient when the cartridge is forced towards the skin. The needle 220 however is rigidly secured to the cartridge body 210 and so continues into the patient's skin. The needle 220 is sufficiently sharp to penetrate through the bellows and on through the skin of the patient, thereby ensuring the sterility of the needle to the point of contact with the patient's skin.

In use, the rupture seal membrane 245 is ruptured as a result of the increase in fluid pressure exerted on it by the medicament 215 when the piston 230 is pushed towards the bung 240. The fluid pressure causes stretching of the rupture seal membrane 245 until the rupture seal membrane 245 reaches breaking point, at which point it ruptures. Once the rupture seal membrane 245 has ruptured, the medicament 215 flows through the aperture of the bung 240 and enters the separation volume 212. As the piston 230 continues to be pushed towards the bung 240, the fluid pressure of the medicament 215 is increased, which causes the medicament 215 to flow out through the needle 220. Movement of the piston 230 towards the bung 240 is the mechanism by which the medicament 215 is expelled from the cartridge 200 and administered to the patient.

There are several advantages associated with the cartridge 200 described above. First, the rupture seal membrane 245 acts as barrier between the medicament 215 and the needle 220 prior to use, but enables fluid connection between the two when administration of the medicament 215 is intended (i.e. by moving the piston 230 and rupturing the rupture seal membrane 245). This prevents prolonged contact between the medicament 215 and the needle 220, which can result in undesired interaction between the needle 220 and the medicament 215. It also allows the auto-injector device to effect administration of the medicament 215 in the usual way (pushing the piston 230), thereby allowing the cartridge 200 to be used with auto-injectors that accommodate standard cartridges, obviating the need to provide a specialised auto-injector device for use with the cartridge 200.

Having the bung 240 and its rupture seal membrane 245 housed within the cartridge body 210 at its front end allows the cartridge 200 to have the shape of a standard medicament cartridge, i.e. having the neck 211 near its front end.

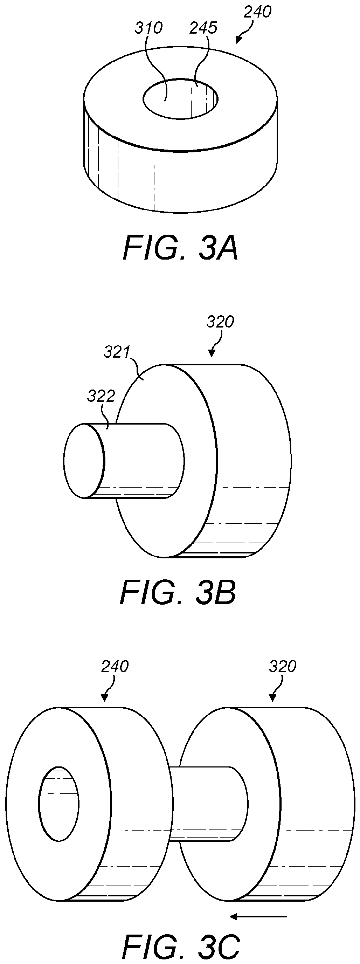

An optional additional feature to the cartridge of Figure 2 will now be described in relation to Figures 3A, 3B and 3C. Figure 3A depicts the bung 240, shown in Figure 2, in more detail. The bung 240 can be made of any suitable material, for instance rubber or plastic. The bung 240 is cylindrical with a circular aperture 310 which runs through its centre. The aperture 310 is closed by a rupture seal membrane 245 which is configured to rupture under fluid pressure only.

Figure 3B shows a piston 320, which is similar to the piston 230 shown in Figure 2, and is positioned within the cartridge 200 in exactly the same way as the piston 230. The main body 321 of the piston 320 is identical to the piston 230 of Figure 2. However the piston 320 further comprises a cylindrical protrusion 322 which extends perpendicularly from the centre of the main body 321 of the piston 320. The protrusion 322 is unitary with the main body of the piston. The piston 320 is a single moulded body comprising the main body 321 and the protrusion 322. However in other embodiments the protrusion is separate to the main body 321 and is attached to it by any suitable means, for example it could be screwed into the main body 321 or attached thereto with adhesive. The bung 240 and the piston 320 have the same diameter. The protrusion 322 is shaped so that it fits within the aperture 310, and advantageously fills the aperture. In the embodiment shown in Figures 3A, 3B and 3C, the aperture 310 and the protrusion 322 have the same diameter so that the protrusion 322 fills the aperture 310.

Figure 3C depicts the bung 240 and the piston 320 when in use. In the Figure, the rupture seal membrane 245 has ruptured, leaving the aperture 310 open. The piston 320 continues to be pushed towards the bung 240, as indicated by the arrow in Figure 3C. This causes the medicament 215 to be expelled through the needle 220 as described previously.

It is desirable that as much medicament as possible is expelled from the cartridge 200, so as to reduce the amount of medicament which remains in the cartridge 200 after use and thus is wasted.

An optimal configuration is one in which the volume which remains between the piston 320 and the septum 225, henceforth referred to as the waste volume, is minimised when the piston 320 is pushed to the point where it abuts the bung 240. The waste volume is equal to the separation volume 212 plus the volume of the aperture 310. The protrusion 322 extends through the aperture 310, thus reducing the waste volume. The length of the protrusion 322 advantageously is equal to, or greater than, the length of the aperture 310. The optimal length of the protrusion 322 is dependent upon the distance between the bung 240 and the needle 220 of Figure 2. To maximum effect, the protrusion 322 occupies as much of the waste volume as possible, without coming into contact with the needle 220. Therefore an optimal length for the protrusion 322 is marginally shorter than the distance between the needle 220 and the proximal end (relative to the needle 220) of the bung 240.

The addition of the protrusion 322 therefore reduces the amount of wasted medicament. Also, the rotational symmetry of the circular aperture 310 positioned centrally through the bung 240 and the cylindrical protrusion 322 positioned centrally on the piston 320, means that the piston 320 and the bung 240 can be placed in any rotational orientation within the cartridge body 210, which simplifies the manufacture of the cartridge 200.

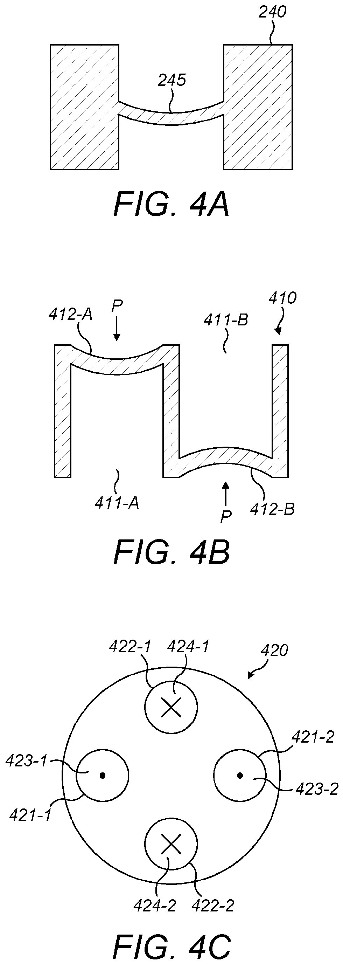

Figure 4A illustrates a possible configuration of the bung 240. The rupture seal membrane 245 is convex in an unstressed position. The rupture seal membrane 245 is curved and is configured to rupture under pressure exerted upon its concave side. The bung 240 is placed within the cartridge body 210 in the correct orientation such that the medicament 215 is in contact with the concave side of the rupture seal membrane 245.

A bung 410, depicted in Figure 4B, comprises two apertures 411-A, 411-B which are sealed by two oppositely orientated rupture seal membranes 412A, 412-B respectively. The rupture seal membranes 412A and 412-B are oppositely orientated in the sense that they are curved in the opposite directions. When viewed from either side of the bung 410, one of the rupture seal membranes 412A, 412-B is convex and the other is concave when in an unstressed position. The rupture seal membrane 412-A ruptures under pressure exerted from above the bung 410 as depicted in Figure 4B, whereas the rupture seal membrane 412-B ruptures under pressure exerted from below. Therefore, the bung 410 can be placed within the cartridge body 210 in place of the bung 240 and in either orientation and still allow the cartridge 200 to function properly. Therefore, the use of the bung 410 simplifies the manufacture of the cartridge 200 because the bung does not need to be correctly orientated (at least not in the longitudinal direction) when inserted.

In an alternative embodiment, a further form for the bung 420 is provided in the cartridge 200, taking the place of the bung 240. A view from above the bung 420 is shown in Figure 4C. The bung comprises four apertures 421-1, 421-2, 422-1 and 422-2. The apertures 421-1 and 421-2 are sealed by rupture seals 423-1 and 423-2, which are convex when viewed from above (as in Figure 4C). The apertures 422-1 and 422-2 are sealed by rupture seals 424-1 and 424-2, which are concave when viewed from above. Therefore apertures 424-1 and 424-2 rupture under fluid pressure exerted from above, whereas apertures 422-1 and 422-2 rupture under fluid pressure exerted from below.

Since the bung 420 comprises rupture seal membranes configured to rupture in each direction, the bung can be fitted in either orientation. Additionally, by having two rupture seal membranes configured to rupture in each direction, the bung 420 is less likely to fail; i.e. if one of the rupture seal membranes were to fail to rupture then the other one should still rupture and ensure the functionality of the cartridge. The bung 420 can therefore be said to reduce the likelihood of the cartridge failing to deliver medicament.

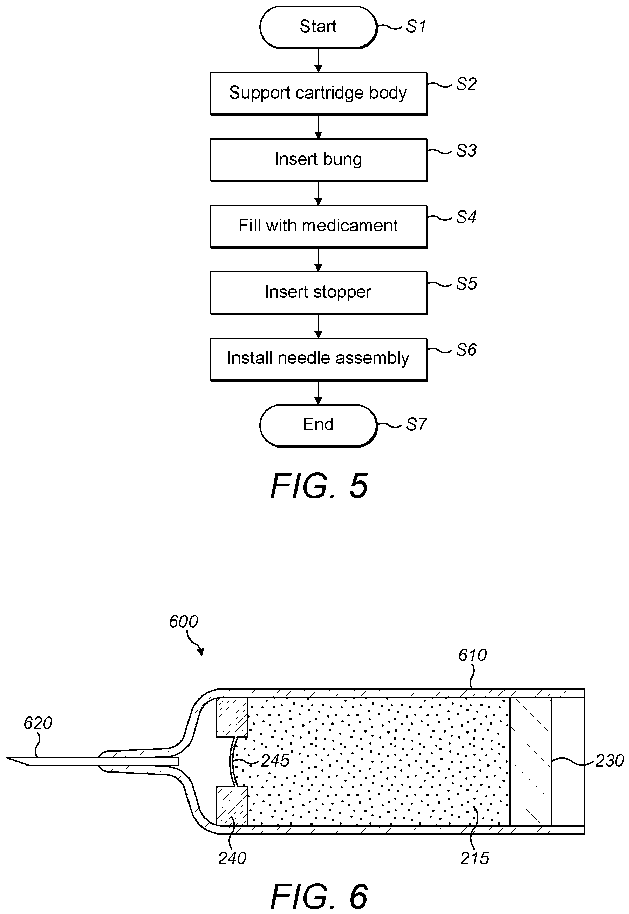

A method of manufacturing the cartridge 200 will now be discussed in relation to Figure 5. The method starts at step S1. At step S2 the cartridge body 210 is supported so that it is held in place securely. Details of the manufacture of the cartridge body 210 are omitted for conciseness.

Next, at step S3, the bung 240 (bung 410 or bung 420 could equally be used) is inserted into the cartridge body 210 from its rear end (i.e. the opposite end to the neck 211). The perimeter of the bung 240 is in complete contact with the inner face of the cartridge body 210. The bung 240 is pushed along the inside of the cartridge body 210 until it reaches the neck 211. The narrower diameter of the neck 211 restricts further movement of the bung 240.

At step S4, the cartridge body 210 is filled with medicament 215 from the rear end of the cartridge body 210. The bung 240 provides a seal near the front end of the cartridge body 210 so that the medicament 215 cannot flow out. When filling the cartridge body with medicament 215, care is taken so as not to provide the medicament with sufficient pressure to rupture the rupture seal membrane 245 of the bung 240.

At step S5 the piston 230 (piston 320 could equally be used) is inserted into the cartridge body 210, sealing the medicament 215 within the cartridge body 210.

The piston 230 may be inserted into the cartridge body 210 under a low pressure environment. This reduces the amount of air trapped inside the cartridge.

At step S6 the needle assembly, which includes the needle 220, the septum 225, the needle holder 250 and bellows 255, is installed at the front end of the cartridge body 210. The method ends at step S7.

Another embodiment of the present disclosure provides a pre-filled syringe 600 as shown in Figure 6. The pre-filled syringe is in some ways similar to the cartridge 200 shown in Figure 2 and described in detail above. Like reference numerals are used for like elements. Only the features of the syringe 600 which distinguish it over the cartridge 200 will be described in detail.

The syringe 600 comprises a hollow housing 610 which is cylindrical with one open end and one closed end. The housing 610 in this embodiment is glass, although plastic could be used in other embodiments. The diameter of the housing 610 is constant along the majority of its length, with the diameter decreasing towards the closed end where the housing 610 is integrally attached to a hollow needle 620. The housing 610 is moulded around the needle 620 such that one end of the needle 620 lies within the housing 610 and the other end lies outside the housing 610.

The bung 240, its rupture seal membrane 245 and the piston 230 function in the same way as in the cartridge 200. Alternatively bung 410 or 420 or piston 320 could be included in the same way as previously described. In this embodiment the bung 240 resides near the closed end of the housing 610, where the diameter of the housing begins to decrease (as seen in Figure 6).

The syringe 600 operates in the same way as the cartridge 200 and can be used in an auto-injector in the same manner as the cartridge 200.

Alternatively, the syringe 600 may be configured for direct use in the administration of the medicament 215 to a patient. In other words the piston 230 is moved by manual force provided by a user, rather than by an auto-injector. There is therefore no need for the syringe 600 to comprise a neck, such as the neck 211, since it does not need to take on a specific shape for use with an auto-injector. As such, the syringe 600 may simply take on a highly convenient shape in terms of manufacture.

Integrally attaching the needle 620 to the housing 610 simplifies the manufacturing process by reducing the number of parts required (i.e. the septum 225 is not required in this embodiment).

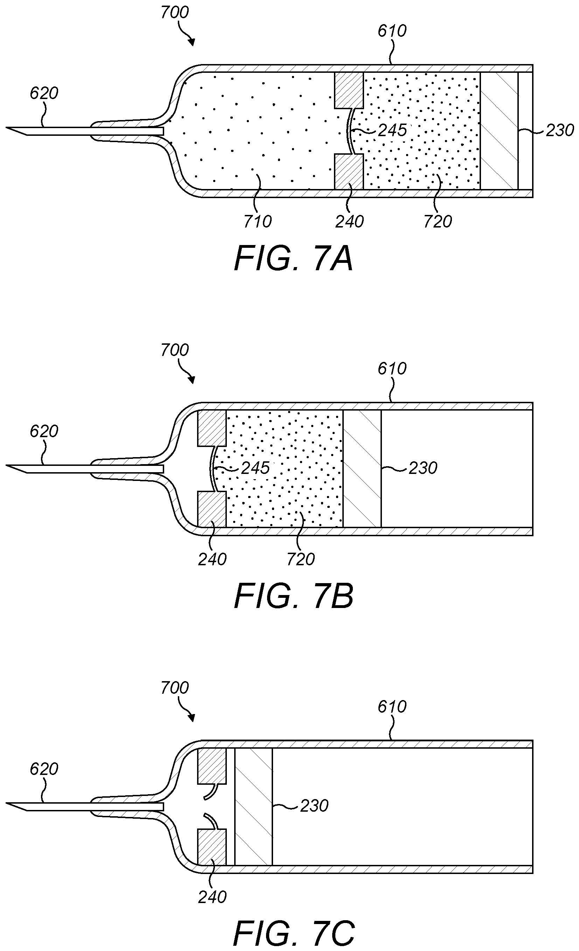

Another alternative embodiment of the present disclosure is the syringe 700, shown in Figure 7. The syringe 700 is similar, in some aspects, to the pre-filled syringe 600 shown in Figure 6. Like reference numerals are used to denote like elements. Only the details of the syringe 700 which are different to the syringe 600 will be described in detail.

The bung 240 is provided part-way along the housing 610 of the syringe 700. The bung 240 separates two different fluids, fluid A 710 and fluid B 720. The bung 240 comprises a rupture seal membrane 245, as in the previously described embodiments. At the distal end of the housing 610, the housing 610 and the bung 240 define a volume in which fluid A 710 is contained. At the proximal of the housing 610, the bung 240, the housing 610 and the piston 230 define a volume in which fluid B 720 is contained.

In operation, a force is applied to the piston 230 in the longitudinal direction towards the needle-end of the syringe 700. The force is transmitted by the incompressible fluid B 720 to the bung 240. The rupture seal membrane 245 is suitably resilient such that the force required to rupture the rupture seal membrane 245 is greater than that required to overcome frictional forces resisting the movement of the bung 240 within the housing 610 and expel fluid A 710 from the syringe 700 via the needle 630. Therefore the force F acting upon the bung 240 causes it to slide along the inside of the housing 610, thereby displacing fluid A 710 out of the syringe via the needle 620.

Once fluid A 710 has been almost fully expelled from the syringe 700, the bung 240 abuts the needle-end of the housing 610 (similarly to the syringe 600 in Figure 6). Further movement of the piston 230 then causes an increase in pressure within fluid B 720 until the pressure is sufficiently high to rupture the rupture seal membrane 245. Once the rupture seal membrane 245 is burst, fluid B 720 is expelled from the syringe 700 via the needle 620 by further movement of the piston 230 towards the needle-end of the housing 610. There may be some mixing of fluids A and B 710, 720 after the rupture seal membrane bursts, although at this stage most of fluid A 710 has been expelled.

In an alternative embodiment similar modifications are made in relation to the cartridge 200.

For example, the manufacture process shown in Fig 5b can be modified to fill the cartridge 200 with two liquids. The first liquid (fluid A) is inserted into the cartridge body 210 between steps S2 and S3 described above. Like the piston 230 described above, the bung 240 is inserted into the housing 610 in a vacuum environment in order to reduce the trapped air volume. The bung 240 may be placed at any position along the length of the cartridge body 210.

The method then continues at step S3 as previously described.

The first liquid (fluid A) may either be a physiologically acceptable "inert" liquid for injection purposes with no active ingredient, e.g., water or saline. In this case, the purpose of the first liquid (fluid A) is to reduce the air volume contained in the separation volume 212.

Alternatively, the first liquid (fluid A) may be a first medicament with an active ingredient. This first medicament, however, is less sensitive to exposure of the needle 220, 620 material. Such a configuration applies for therapy cases in which two separate medicaments have to be delivered to the same injection site without mixing these medicaments prior to the injection.

The second liquid (fluid B) is medicament 215 and is disposed between the bung 240 and the piston 230 (as described above).

In this way, the syringe 700 allows injection of two different fluids sequentially through the same needle without mixing the fluids prior to or during the injection.

Although in the above the bungs 240, 410, 420 and the pistons 230, 320 are circular, in embodiments where the cartridge body 210 or syringe housing 710 have a non-circular cross-sectional shape (e.g. oval, rectangle, triangle etc.) then the bung and the piston are provided with a corresponding cross-sectional shape.

Additionally, although the embodiments described above include a needle that is fixedly attached to the cartridge or syringe, in some embodiments the needle may be provided separately. In such embodiments, the needle might be attached by the user for example. Also if the cartridge or syringe is reusable, or fixed-dose, then the user will often need to replace the needle between doses.

Also, the rupture seal membranes 245, 412-A, 412-B, 423-1, 423-2, 424-1, 424-2 and the apertures 310, 411-A, 411-B, 421-1, 421-2, 422-1, 422-2 are circular in the above, but in other embodiments they are shaped differently, for instance oval-shaped, square, triangular etc.

Accordingly, in embodiments where the aperture 310 is non-circular, a correspondingly-shaped protrusion 322 can be provided on the piston. For example, if the aperture 310 is square then the protrusion 322 is a square-prism.

Furthermore in some embodiments the apertures 310, 411-A, 411-B, 421-1, 421-2, 422-1, 422-2 may be offset from the centre of the bung. In these embodiments the protrusion 322, if provided, is also offset from the centre of the piston so that it can coincide with the aperture 310.

Although in the above the rupture seal membrane 245 is integral with the bung 240, in other embodiments the rupture seal membrane 245 may be provided separately to seal the aperture 310 of the bung 240.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The term "drug delivery device" shall encompass any type of device or system configured to dispense a drug or medicament into a human or animal body. Without limitation, a drug delivery device may be an injection device (e.g., syringe, pen injector, auto injector, large-volume device, pump, perfusion system, or other device configured for intraocular, subcutaneous, intramuscular, or intravascular delivery), skin patch (e.g., osmotic, chemical, micro-needle), inhaler (e.g., nasal or pulmonary), an implantable device (e.g., drug- or API-coated stent, capsule), or a feeding system for the gastro-intestinal tract. The presently described drugs may be particularly useful with injection devices that include a needle, e.g., a hypodermic needle for example having a Gauge number of 24 or higher.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refer to any substance which is sufficiently structurally similar to the original substance so as to have substantially similar functionality or activity (e.g., therapeutic effectiveness). In particular, the term "analogue" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir®); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba®); B29-N-(N-lithocholyl-gamma-glutamyl)-des(B30) human insulin; B29-N-(ω-carboxyheptadecanoyl)-des(B30) human insulin and B29-N-(ω-carboxyhepta¬decanoyl) human insulin.

Examples of GLP-1, GLP-1 analogues and GLP-1 receptor agonists are, for example, Lixisenatide (Lyxumia®, Exenatide (Exendin-4, Byetta®, Bydureon®, a 39 amino acid peptide which is produced by the salivary glands of the Gila monster), Liraglutide (Victoza®), Semaglutide, Taspoglutide, Albiglutide (Syncria®), Dulaglutide (Trulicity®), rExendin-4, CJC-1134-PC, PB-1023, TTP-054, Langlenatide/ HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An example of an oligonucleotide is, for example: mipomersen sodium (Kynamro®), a cholesterol-reducing antisense therapeutic for the treatment of familial hypercholesterolemia.

Examples of DPP4 inhibitors are Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc®), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigens. The antibody can be polyclonal, monoclonal, recombinant, chimeric, de-immunized or humanized, fully human, non-human, (e.g., murine), or single chain antibody. In some embodiments, the antibody has effector function and can fix a complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope and spirit of the present invention, which encompass such modifications and any and all equivalents thereof.

## Claims

1. Injection apparatus comprising:
a hollow body (210) having a first end at which a hollow needle (220) is or can be located;
a bung (240) with a rupture seal membrane (245) located within the hollow body (210); and
a piston (230) located within the hollow body (210), wherein the piston (230), the hollow body (210) and the bung (240) define a volume containing a liquid medicament (215), wherein the bung (240) is nearer than the piston (230) to the first end of the hollow body (210), and wherein the rupture seal membrane (245) initially isolates the needle (220) from the medicament (215),
wherein the rupture seal membrane (245) is configured to rupture through fluid pressure alone without the rupture seal membrane (245) contacting the needle (220) thereby to allow the medicament (215) to be expelled through the needle (220) upon movement of the piston (230) causing the provision of pressure to the medicament (215), and wherein the piston (230) comprises a protrusion (322) located on the side of the piston (230) which faces the first end of the hollow body (210), the protrusion (322) shaped so as to extend into an aperture (310) in the bung (240) at the end of medicament expulsion.

2. The injection apparatus of claim 1, wherein the aperture (310) in the bung (240) is circular and wherein the protrusion (322) of the piston (230) is cylindrical in shape.

3. The injection apparatus of claim 2, wherein the aperture (310) in the bung (240) lies on the longitudinal axis of the bung (240) and wherein the protrusion (322) of the piston (230) lies on the longitudinal axis of the piston (230).

4. The injection apparatus of claim 2 or claim 3, wherein a diameter of the aperture (310) in the bung (240) is the same as an outside diameter of the protrusion (322).

5. The injection apparatus of any of claims 2 to 4, wherein a length of the protrusion (322) is the same or substantially the same as a length of the aperture (310) in the bung (240).

6. The injection apparatus of any preceding claim, wherein the needle (220) is connected at the first end of the hollow body (210) and wherein an end of the needle (220) that is internal to the hollow body (210) is nearer than the rupture seal membrane (245) to the first end of the hollow body (210).

7. The injection apparatus of any preceding claim, wherein the one or more rupture seal membranes (245) are convex in an unstressed position.

8. The injection apparatus of any preceding claim, wherein the injection apparatus is a medicament cartridge (200) for use in an injector.

9. The injection apparatus according to any preceding claim, wherein a liquid substance is disposed between the first end of the hollow body (210) and the bung (240).

10. The injection apparatus of any preceding claim, wherein the hollow body includes a neck and wherein the bung (240) is located against the neck (211).

11. The injection apparatus of any of claims 1 to 7, wherein the injection apparatus is a syringe device and wherein the hollow body (210) forms the outer housing of the syringe device.

12. The injection apparatus of claim 11, wherein the bung (240) is located against the first end of the hollow body (210).

13. A method of constructing an injection apparatus, the method comprising:
supporting a hollow body (210), the hollow body (210) comprising a front end configured to house a hollow needle (220) and a rear end which is open;
inserting a bung (240) into the hollow body (210) at its rear end, wherein the bung (240) comprises a rupture seal membrane (245) configured to rupture through fluid pressure alone;
pushing the bung (240) along the hollow body (210) towards its front end, the bung (240) forming a seal with an inner wall of the hollow body (210);
providing the hollow body (210) with a liquid medicament (215);
inserting a piston (230) into the hollow body (210) at its rear end, wherein the piston (230) comprises a protrusion (322) located on the side of the piston (230) which faces the first end of the hollow body (210), the protrusion (322) shaped so as to extend into an aperture (310) in the bung (240) at the end of medicament expulsion; and
pushing the piston (230) along the hollow body (210) towards the bung (240), until the piston (230) reaches the medicament, wherein the piston (230) forms a seal with the inner wall of the hollow body (210) thereby sealing the medicament in the volume defined by the piston (230), the hollow body (210) and the bung (240).

14. The method of claim 13, wherein the aperture (310) in the bung (240) is circular and wherein the protrusion (322) of the piston (230) is cylindrical in shape.

15. The method of claim 14, wherein the aperture (310) in the bung (240) lies on the longitudinal axis of the bung (240) and wherein the protrusion (322) of the piston (230) lies on the longitudinal axis of the piston (230).

## Patentansprüche

1. Injektionsvorrichtung, aufweisend:
einen hohlen Körper (210) mit einem ersten Ende, an dem eine Hohlnadel (220) ist oder angeordnet werden kann,
einen Pfropfen (240) mit einer Berstdichtungsmembran (245), die in dem hohlen Körper (210) angeordnet ist, und
einen in dem hohlen Körper (210) angeordneten Kolben (230), wobei der Kolben (230), der hohle Körper (210) und der Pfropfen (240) ein Volumen definieren, das ein flüssiges Medikament (215) enthält, wobei der Pfropfen (240) dem ersten Ende des hohlen Körpers (210) näher ist als der Kolben (230) und wobei die Berstdichtungsmembran (245) die Nadel (220) anfänglich von dem Medikament (215) isoliert,
wobei die Berstdichtungsmembran (245) dazu ausgestaltet ist, allein durch Fluiddruck zum Bersten gebracht zu werden, ohne dass die Berstdichtungsmembran (245) die Nadel (220) kontaktiert, wodurch gestattet wird, dass das Medikament (215) bei Bewegung des Kolbens (230), wodurch das Medikament (215) mit Druck beaufschlagt wird, durch die Nadel (220) ausgestoßen wird, und wobei der Kolben (230) einen Vorsprung (322) aufweist, der an der Seite des Kolbens (230) angeordnet ist, die dem ersten Ende des hohlen Körpers (210) zugewandt ist, wobei der Vorsprung (322) so gestaltet ist, dass er sich in eine Öffnung (310) in dem Pfropfen (240) am Ende des Medikamentausstoßes erstreckt.

2. Injektionsvorrichtung nach Anspruch 1, wobei die Öffnung (310) in dem Pfropfen (240) kreisförmig ist und wobei der Vorsprung (322) des Kolbens (230) zylinderförmig ist.

3. Injektionsvorrichtung nach Anspruch 2, wobei die Öffnung (310) in dem Pfropfen (240) auf der Längsachse des Pfropfens (240) liegt und wobei der Vorsprung (322) des Kolbens (230) auf der Längsachse des Kolbens (230) liegt.

4. Injektionsvorrichtung nach Anspruch 2 oder Anspruch 3, wobei ein Durchmesser der Öffnung (310) in dem Pfropfen (240) derselbe wie ein äußerer Durchmesser des Vorsprungs (322) ist.

5. Injektionsvorrichtung nach einem der Ansprüche 2 bis 4, wobei eine Länge des Vorsprungs (322) dieselbe oder im Wesentlichen dieselbe wie eine Länge der Öffnung (310) in dem Pfropfen (240) ist.

6. Injektionsvorrichtung nach einem der vorhergehenden Ansprüche, wobei die Nadel (220) an dem ersten Ende des hohlen Körpers (210) verbunden ist und wobei ein Ende der Nadel (220), das innerhalb des hohlen Körpers (210) liegt, dem ersten Ende des hohlen Körpers (210) näher ist als die Berstdichtungsmembran (245).

7. Injektionsvorrichtung nach einem der vorhergehenden Ansprüche, wobei die eine oder die mehreren Berstdichtungsmembranen (245) in einer entspannten Position konvex sind.

8. Injektionsvorrichtung nach einem der vorhergehenden Ansprüche, wobei die Injektionsvorrichtung eine Medikamentkartusche (200) zur Verwendung in einem Injektor ist.

9. Injektionsvorrichtung nach einem der vorhergehenden Ansprüche, wobei eine flüssige Substanz zwischen dem ersten Ende des hohlen Körpers (210) und dem Pfropfen (240) angeordnet ist.

10. Injektionsvorrichtung nach einem der vorhergehenden Ansprüche, wobei der hohle Körper einen Hals aufweist und wobei der Pfropfen (240) an dem Hals (211) angeordnet ist.

11. Injektionsvorrichtung nach einem der Ansprüche 1 bis 7, wobei die Injektionsvorrichtung eine Spritzenvorrichtung ist und wobei der hohle Körper (210) das äußere Gehäuse der Spritzenvorrichtung bildet.

12. Injektionsvorrichtung nach Anspruch 11, wobei der Pfropfen (240) an dem ersten Ende des hohlen Körpers (210) angeordnet ist.

13. Verfahren zum Konstruieren einer Injektionsvorrichtung, wobei das Verfahren Folgendes aufweist:
Stützen eines hohlen Körpers (210), wobei der hohle Körper (210) ein vorderes Ende, das zur Unterbringung einer Hohlnadel (220) ausgestaltet ist, und ein hinteres Ende, das offen ist, aufweist,
Einführen eines Pfropfens (240) in den hohlen Körper (210) an seinem hinteren Ende, wobei der Pfropfen (240) eine Berstdichtungsmembran (245) aufweist, die dazu ausgestaltet ist, allein durch Fluiddruck zum Bersten gebracht zu werden,
Schieben des Pfropfens (240) entlang des hohlen Körpers (210) zu seinem vorderen Ende hin, wobei der Pfropfen (240) eine Dichtung mit einer inneren Wand des hohlen Körpers (210) bildet,
Versehen des hohlen Körpers (210) mit einem flüssigen Medikament (215),
Einführen eines Kolbens (230) in den hohlen Körper (210) an seinem hinteren Ende, wobei der Kolben (230) einen Vorsprung (322) aufweist, der an der Seite des Kolbens (230) angeordnet ist, die dem ersten Ende des hohlen Körpers (210) zugewandt ist, wobei der Vorsprung (322) so gestaltet ist, dass er sich in eine Öffnung (310) in dem Pfropfen (240) am Ende des Medikamentausstoßes erstreckt, und
Schieben des Kolbens (230) entlang des hohlen Körpers (210) zu dem Pfropfen (240) hin, bis der Kolben (230) das Medikament erreicht, wobei der Kolben (230) eine Dichtung mit der inneren Wand des hohlen Körpers (210) bildet, wodurch das Medikament in dem durch den Kolben (230), den hohlen Körper (210) und den Pfropfen (240) definierten Volumen abgedichtet wird.

14. Verfahren nach Anspruch 13, wobei die Öffnung (310) in dem Pfropfen (240) kreisförmig ist und wobei der Vorsprung (322) des Kolbens (230) zylinderförmig ist.

15. Verfahren nach Anspruch 14, wobei die Öffnung (310) in dem Pfropfen (240) auf der Längsachse des Pfropfens (240) liegt und wobei der Vorsprung (322) des Kolbens (230) auf der Längsachse des Kolbens (230) liegt.

## Revendications

1. Appareil d'injection comprenant :
un corps creux (210) ayant une première extrémité au niveau de laquelle une aiguille creuse (220) est ou peut être située ;
un bouchon (240) avec une membrane d'étanchéité frangible (245) située à l'intérieur du corps creux (210) ; et
un piston (230) situé à l'intérieur du corps creux (210), le piston (230), le corps creux (210) et le bouchon (240) définissant un volume contenant un médicament liquide (215), le bouchon (240) étant plus près que le piston (230) de la première extrémité du corps creux (210), et la membrane d'étanchéité frangible (245) isolant initialement l'aiguille (220) du médicament (215),
la membrane d'étanchéité frangible (245) étant configurée pour se rompre sous l'effet seulement de la pression de fluide sans que la membrane d'étanchéité frangible (245) ne vienne en contact avec l'aiguille (220), pour ainsi permettre au médicament (215) d'être expulsé à travers l'aiguille (220) du fait du mouvement du piston (230) provoquant la génération de pression dans le médicament (215), et le piston (230) comprenant une saillie (322) située du côté du piston (230) tourné vers la première extrémité du corps creux (210), la saillie (322) étant formée de manière à s'étendre dans une ouverture (310) dans le bouchon (240) à la fin de l'expulsion du médicament.

2. Appareil d'injection selon la revendication 1, dans lequel l'ouverture (310) dans le bouchon (240) est circulaire et dans lequel la saillie (322) du piston (230) a une forme cylindrique.

3. Appareil d'injection selon la revendication 2, dans lequel l'ouverture (310) dans le bouchon (240) est située sur l'axe longitudinal du bouchon (240) et dans lequel la saillie (322) du piston (230) est située sur l'axe longitudinal du piston (230) .

4. Appareil d'injection selon la revendication 2 ou la revendication 3, dans lequel un diamètre de l'ouverture (310) dans le bouchon (240) est identique à un diamètre extérieur de la saillie (322) .

5. Appareil d'injection selon l'une quelconque des revendications 2 à 4, dans lequel une longueur de la saillie (322) est identique ou sensiblement identique à une longueur de l'ouverture (310) dans le bouchon (240).

6. Appareil d'injection selon l'une quelconque des revendications précédentes, dans lequel l'aiguille (220) est connectée à la première extrémité du corps creux (210) et dans lequel une extrémité de l'aiguille (220) qui est interne au corps creux (210) est plus près que la membrane d'étanchéité frangible (245) de la première extrémité du corps creux (210).

7. Appareil d'injection selon l'une quelconque des revendications précédentes, dans lequel la ou les membranes d'étanchéité frangibles (245) sont convexes dans une position non sollicitée.

8. Appareil d'injection selon l'une quelconque des revendications précédentes, l'appareil d'injection étant une cartouche de médicament (200) pour l'utilisation dans un injecteur.

9. Appareil d'injection selon l'une quelconque des revendications précédentes, dans lequel une substance liquide est disposée entre la première extrémité du corps creux (210) et le bouchon (240) .

10. Appareil d'injection selon l'une quelconque des revendications précédentes, dans lequel le corps creux comporte un col et dans lequel le bouchon (240) est situé contre le col (211).

11. Appareil d'injection selon l'une quelconque des revendications 1 à 7, l'appareil d'injection étant un dispositif de seringue et le corps creux (210) formant le boîtier extérieur du dispositif de seringue.

12. Appareil d'injection selon la revendication 11, dans lequel le bouchon (240) est situé contre la première extrémité du corps creux (210).

13. Procédé de construction d'un appareil d'injection, le procédé comprenant les étapes consistant à :
supporter un corps creux (210), le corps creux (210) comprenant une extrémité avant configurée pour recevoir une aiguille creuse (220) et une extrémité arrière qui est ouverte ;
insérer un bouchon (240) dans le corps creux (210) au niveau de son extrémité arrière, le bouchon (240) comprenant une membrane d'étanchéité frangible (245) configurée pour se rompre sous l'effet seulement de la pression de fluide ;
pousser le bouchon (240) le long du corps creux (210) vers son extrémité avant, le bouchon (240) formant un joint avec une paroi interne du corps creux (210) ;
fournir au corps creux (210) un médicament liquide (215) ;
insérer un piston (230) dans le corps creux (210) au niveau de son extrémité arrière, le piston (230) comprenant une saillie (322) située du côté du piston (230) tourné vers la première extrémité du corps creux (210), la saillie (322) étant formée de manière à s'étendre dans une ouverture (310) dans le bouchon (240) à la fin de l'expulsion du médicament ; et
pousser le piston (230) le long du corps creux (210) vers le bouchon (240), jusqu'à ce que le piston (230) atteigne le médicament, le piston (230) formant un joint avec la paroi interne du corps creux (210) pour ainsi sceller le médicament dans le volume défini par le piston (230), le corps creux (210) et le bouchon (240).

14. Procédé selon la revendication 13, dans lequel l'ouverture (310) dans le bouchon (240) est circulaire et dans lequel la saillie (322) du piston (230) a une forme cylindrique.

15. Procédé selon la revendication 14, dans lequel l'ouverture (310) dans le bouchon (240) est située sur l'axe longitudinal du bouchon (240) et dans lequel la saillie (322) du piston (230) est située sur l'axe longitudinal du piston (230).
